(19) Europäisches Patentamt

European Patent Office

Office européen des brevets

(11) **EP 1 566 227 B1**

## (12) EUROPÄISCHE PATENTSCHRIFT

(45) Veröffentlichungstag und Bekanntmachung des Hinweises auf die Patenterteilung:
**26.04.2006 Patentblatt 2006/17**

(51) Int Cl.:
***B21B 38/02*** *(2006.01)*

(21) Anmeldenummer: **04029404.3**

(22) Anmeldetag: **11.12.2004**

(54) **Verfahren zur Ermittlung von Planheitsmessfehlern in Bändern, insbesondere Stahl- und Metallbändern, und Planheitsmessrolle**

Method for measuring flatness anomalies in strip materials, especially steel strip and metal strip and flatness measuring roll

Procédé de détection des défauts de planéité dans des bandes, en particulier des bandes en acier et des bandes métalliques et rouleau de détection de la planéité

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **20.02.2004 DE 102004008303**

(43) Veröffentlichungstag der Anmeldung:
**24.08.2005 Patentblatt 2005/34**

(73) Patentinhaber: **BWG BERGWERK- UND WALZWERK-MASCHINENBAU GMBH D-47051 Duisburg (DE)**

(72) Erfinder: **Noé, Andreas, Dr. mont., Dipl.-Ing. 47647 Kerken (DE)**

(74) Vertreter: **Albrecht, Rainer Harald et al Patentanwälte Andrejewski, Honke & Sozien, Postfach 10 02 54 45002 Essen (DE)**

(56) Entgegenhaltungen:
**EP-A- 0 982 563          EP-A- 1 369 186 WO-A- 02/070154          US-A1- 2002 092 365**

## Beschreibung

Die Erfindung betrifft ein Verfahren zur Ermittlung von Planheitsfehlern in Bändern, insbesondere Stahl- und Metallbändern beim z. B. Durchlaufen von Bandprozesslinien oder Walzstraßen.

Zur Durchführung eines solchen Verfahrens ist beispielsweise aus EP 1 369 186 A2 eine Planheitsmessrolle mit zumindest einem integrierten sich über die Rollenlänge wendelförmig oder gerade erstreckenden Messbalken zum Ermitteln von auf das die Messrolle teilweise umschlingende Band einwirkenden Zugkräften bekannt, wobei der Messbalken während der Zugkraftermittlung mit seinem Anfangsbereich und seinem Endbereich zumindest teilweise innerhalb des von dem Band gebildeten Umschlingungswinkels angeordnet und endseitig auf Kraftmessdosen bzw. Kraftmessgebern aufgelagert ist. Der Messbalken ist mit Hilfe von zwei Vorspannschrauben am Rollenkörper befestigt, die durch Innenbohrungen der Kraftmessgeber hindurch geführt sind und die Kraftmessgeber mit einer vorgegebenen Vorspannkraft beaufschlagen. Derartige Maßnahmen kennt man auch bei Verwirklichung radial belasteter und kreisringförmiger Abdeckungen für die Kraftmessgeber (vgl. DE 42 36 657 C2).

Bei den Kraftmessgebern handelt es sich um Piezo-Quarze, die eine Vorspannung verlangen, um Druckkräfte messen zu können. Die sie durchdringenden Vorspannschrauben erzeugen jedoch zwangsläufig einen Kraftnebenschluss, so dass die Messkräfte nicht komplett in die Kraftmessgeber, sondern auch teilweise in die Vorspannschrauben eingeleitet werden. Der Kraftnebenschluss kann bis zu 30 % betragen. Zwar lässt sich der Einfluss des Kraftnebenschlusses zunächst durch eine Kalibrierung gleichsam herausfiltern, das aber setzt voraus, dass der Kraftnebenschluss konstant bleibt. Tatsächlich besteht jedoch die Gefahr, dass sich die Größe des Kraftnebenschlusses im Betrieb, z. B. aufgrund von Temperaturänderungen ändert und somit die Messergebnisse verfälscht. Außerdem entsprechen dann die Messsignale nicht der vollständigen Messkraft. Zwar könnte man daran denken, als Kraftmessgeber Dehnmessstreifen einzusetzen, die ohne Vorspannung auskommen, so dass kein Kraftnebenschluss erzeugt wird. Solche Dehnmessstreifen weisen jedoch im Vergleich mit Piezo-Quarzen eine verhältnismäßig geringe Messgenauigkeit auf.

Im Rahmen der US 2002/0092365 A1 sowie der WO 02/070154 A1 werden Planheitsmessrollen vorgestellt, die über mehrere über den Rollenmantel verteilt angeordnete Messwertgeber verfügen. Dabei besteht unverändert das Problem, das aufgrund der Führung der Messwertgeber im Rollenmantel ein Kraftnebenschluss beobachtet wird und demzufolge die Messwerte eine Verfälschung erfahren.

Der Erfindung liegt das technische Problem zu Grunde, ein Verfahren und eine Planheitsmessrolle zur Ermittlung von Planheitsfehlern in Bändern, insbesondere Stahl- und Metallbändern, anzugeben, wonach sich Piezo-Quarze als Kraftmessgeber ohne nachteiligen Kraftnebenschluss einsetzen lassen.

Zur Lösung dieser Aufgabe ist Gegenstand der Erfindung ein Verfahren zur Ermittlung von Planheitsmessfehlern in Bändern, insbesondere Stahl- und Metallbändern, im Wege einer Messung der Bandzugsspannungsverteilung über die Bandbreite, wobei das über die gesamte Bandbreite unter Zugspannung stehende Band eine Planheitsmessrolle mit einem vorgegebenen Umschlingungswinkel umschlingt und dadurch örtliche Anpresskräfte entsprechend der örtlichen Längs-Zugspannungsverteilung in Bandbreitenrichtung auf die Planheitsmessrolle ausübt, aus denen sich die Bandzugspannungsverteilung erfassen lässt, wobei die Planheitsmessrolle zumindest einen Messkörper mit zwei um 180° versetzten in den Rollenmantel integrierten und auf Kraftmessgebern abgestützten Teilmessgebern aufweist, die von dem Rollenmantel durch einen umlaufenden Bewegungsspalt entkoppelt und über zumindest eine Zugstange miteinander verspannt sind, wobei ferner die Messwerte der Kraftmessgeber eines jeden Teilmesskörpers aufsummiert werden, dann die Summenwerte der beiden Teilmesskörper voneinander subtrahiert werden und der dadurch gebildete Differenzmesswert der gesamten örtlichen Anpresskraft abzüglich der Gewichtskraft des Messkörpers bzw. seiner Teilmesskörper entspricht. - Diese Maßnahmen der Erfindung haben zur Folge, dass die örtlichen Anpresskräfte vollständig ohne Kraftnebenschluss gemessen werden. Tatsächlich zeichnet sich das erfindungsgemäße Verfahren durch hohe Messgenauigkeit unter Berücksichtigung eines optimierten dynamischen Ansprechverhaltens bei hinreichender Steifigkeit der Kraftmessgeber aus. Die Fliehkräfte der beiden Teilmesskörper, die bei hohen Rollenumdrehungszahlen die Größenordnung der Messkräfte erreichen können, kompensieren sich aufgrund der Tatsache heraus, dass die beiden Teilmesskörper um 180° versetzt zueinander angeordnet sind. Ferner besteht die Möglichkeit, die Gewichtskraft des Messkörpers in einer Kalibriermessung als Funktion des Rollendrehwinkels ohne Anpresskräfte zu ermitteln und als Korrekturfunktion für die Messwertauswertung heranzuziehen. Die Gewichtskraftkomponente beschreibt über den Drehwinkel der Messrolle, der mitgemessen wird, eine Sinusfunktion. Durch eine einmalige Kalibrierung kann eine Korrekturfunktion als Funktion des Drehwinkels bestimmt werden, so dass der Gewichtseinfluss auf die Messung neutralisiert wird. Somit lassen sich die Messkräfte bei einem bestimmten Drehwinkel durch die Messsignale der Kraftmessgeber vollständig bestimmen. Tatsächlich entspricht bei der Messung mit Band das korrigierte Messsignal vollständig der örtlichen Anpresskraft.

Gegenstand der Erfindung ist ferner eine Planheitsmessrolle zur Durchführung des erfindungsgemäßen Verfahrens und folglich zur Ermittlung von Planheitsfehlern in Bändern, insbesondere Stahlbändern und Metallbändern beim z. B. Durchlaufen von Bandprozesslinien oder Walzstraßen, mit zumindest einen Messkörper mit zwei um 180° versetzten in den Rollenmantel integrierten und auf Kraftmessgebern abgestützten Teilmesskörpern, die von dem Rollenmantel durch einen umlaufenden Bewegungsspalt entkoppelt und über zumindest eine Zugstange miteinander verspannt sind, wobei im Zuge einer Messung der Bandzugspannungsverteilung über die Bandbreite das über die gesamte Bandbreite unter Zugspannung stehende Band die Planheitsmessrolle mit einem vorgegebenen Umschlingungswinkel umschlingt und dadurch örtliche Anpresskräfte entsprechend der örtlichen Längs-Zugspannungsverteilung in Bandbreitenrichtung auf die Planheitsmessrolle ausübt, aus denen sich die Bandzugspannungsverteilung ermitteln lässt. Um die Messkraft korrekt und vollständig zu erfassen, wird zunächst die Summe der Messsignale der Kraftmessgeber an dem Teilmesskörper im Bereich der Bandumschlingung gebildet. Davon ist die Summe der Messsignale der Kraftmessgeber am gegenüberliegenden Teilmesskörper sowie das Gewicht des Messkörpers und folglich der beiden Teilmesskörper zu subtrahieren. Die Teilmesskörper können reißförmig ausgebildet und jeweils zentral auf einen Kraftmessgeber abgestützt sein. Nach bevorzugter Ausführungsform der Erfindung ist jedoch vorgesehen, dass die Teilmesskörper als Messbalken ausgebildet und in den Balkenendbereichen auf Kraftmessgebern abgestützt sind. Die Kraftmessgeber sind als Piezo-Quarze ausgebildet. Die Messbalken können gerade oder schräg zur Rollenachse angeordnet sein. Aus der Schräganordnung resultiert ein wendelförmiger Verlauf der Messbalken. In den die Teilmesskörper umgebenden Bewegungsspalt kann ein dauerelastischer Kleber und/oder eine Dichtung - bei kreisförmiger Ausführungsform der Messkörper ein O-Ring eingesetzt sein. Zweckmäßigerweise weisen die Messkörper mehr als zwei Teilmesskörper auf, wodurch sich die Anzahl der Messungen pro Rollenumdrehung erhöht. Die Messkörper bzw. ihre Teilmesskörper sind vorzugsweise von einer Metallfolie abgedeckt, die mit der Rollenoberfläche adhäsiv verbunden ist, um Scherbeanspruchungen in den angepressten Bandbereichen zu vermeiden. Das gilt im Übrigen auch für den Fall, dass die erfindungsgemäße Planheitsmessrolle eine Ummantelung aus Gummi, Kunststoff oder Hartmetall aufweist. Endlich ist vorgesehen, dass zwei oder mehr Messkörper im jeweiligen Bandbreitenbereich angeordnet sind, wodurch sich die Anzahl der Messungen pro Rollenumdrehung ebenfalls erhöht.

Im Folgenden wird die Erfindung anhand einer lediglich ein Ausführungsbeispiel darstellenden Zeichnung näher erläutert. Es zeigen:

Fig. 1    eine Planheitsmessrolle in schematischer Draufsicht mit Messbalken als Teilmesskörper,

Fig. 2    einen Schnitt A-A durch den Gegenstand nach Fig. 1 mit in die Schnittebene eingeschränkter unterer Bildhälfte,

Fig. 3    eine abgewandelte Ausführungsform des Gegenstandes nach Fig. 1 mit kreisförmigen Teilmesskörpern,

Fig. 4    einen Radialschnitt durch den Gegenstand nach Fig. 3 im Bereich der Teilmesskörper und

Fig. 5    ein Kräfteschaubild für den Gegenstand nach Fig. 2.

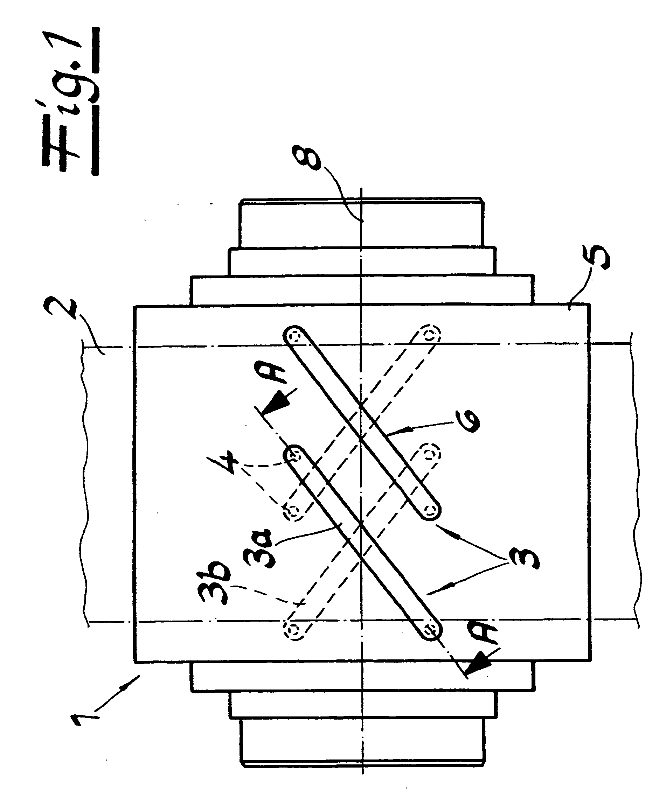

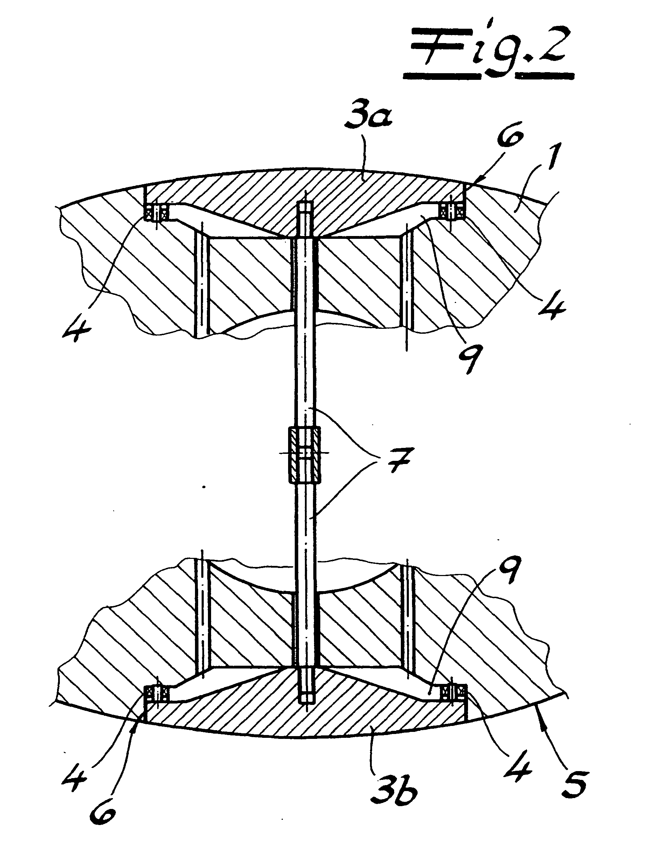

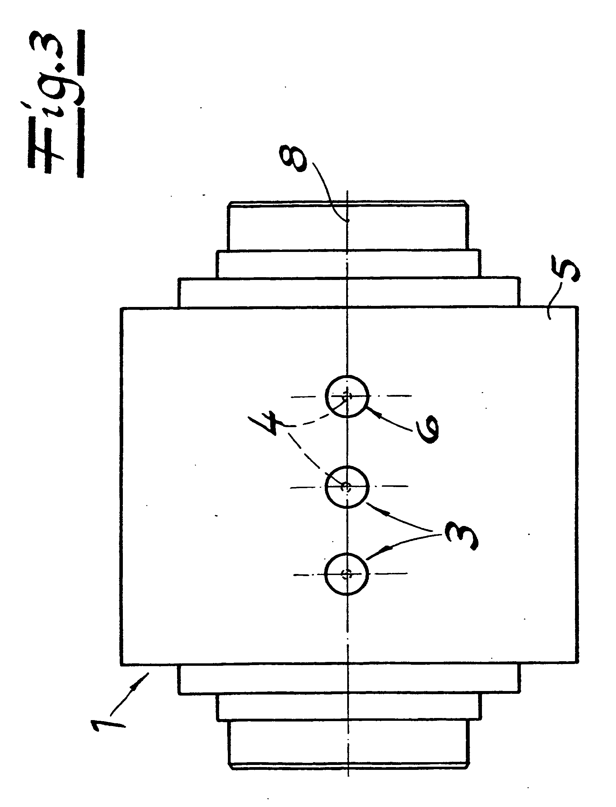

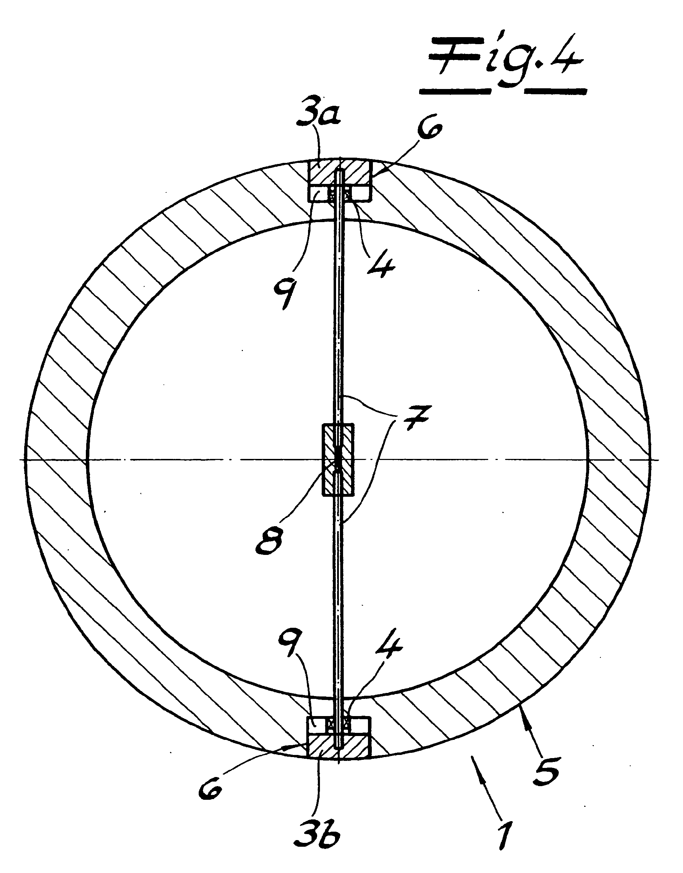

In den Figuren 1 bis 4, ist eine Planheitsmessrolle 1 zur Ermittlung von Planheitsfehlern in Bändern 2, insbesondere Stahlbändern und Metallbändern beim beispielsweise Durchlaufen von Bandprozesslinien oder Walzwerkstraßen dargestellt. Diese Planheitsmessrolle weist zumindest einen Messkörper 3 mit zwei um 180° versetzten in den Rollenmantel integrierten und auf Kraftmessgebern 4 abgestützten Teilmesskörpern 3a, 3b auf, die von dem Rollenmantel 5 durch einen umlaufenden Bewegungsspalt 6 entkoppelt und über zumindest eine Zugstange 7 miteinander verspannt sind, wobei im Zuge einer Messung der Bandzugspannungsverteilung über die Bandbreite das über die gesamte Bandbreite unter Zugspannung stehende Band 2 die Planheitsmessrolle 1 mit einem vorgegebenen Umschlingungswinkel umschlingt und dadurch örtliche Anpresskräfte entsprechend der örtlichen Längszugspannungsverteilung in Bandbreitenrichtung auf die Planheitsmessrolle 1 ausübt, aus denen sich die Bandzugspannungsverteilung ermitteln lässt. Bei der Ausführungsform nach den Figuren 1 und 2 sind die Teilmesskörper 3a, 3b als Messbalken ausgebildet und in den Balkenendbereichen auf Kraftmessgebern 4 abgestützt. Bei der Ausführungsform nach den Figuren 3 und 4 sind die Teilmesskörper 3a, 3b kreisförmig ausgebildet und jeweils zentral auf einem Kraftmessgeber 4 abgestützt. Die Kraftmessgeber 4 sind als Piezo-Quarze ausgebildet.

Bei der Ausführungsform mit den Messbalken sind diese Messbalken schräg zur Rollenachse 8 angeordnet und nehmen folglich einen wendelförmigen Verlauf an. Vorzugsweise sind zwei oder mehr Messkörper 3 im jeweiligen Bandbreitenbereich angeordnet. Zur Aufnahme der Messkörper 3 bzw. ihrer Teilmesskörper weist der Rollenkörper Ausnehmungen 9 auf, wobei die Teilmesskörper 3a, 3b mit der Rollenoberfläche fluchten und einen extrem engen Bewegungsspalt 6 mit der sie umgebenden Ausnehmung bilden.

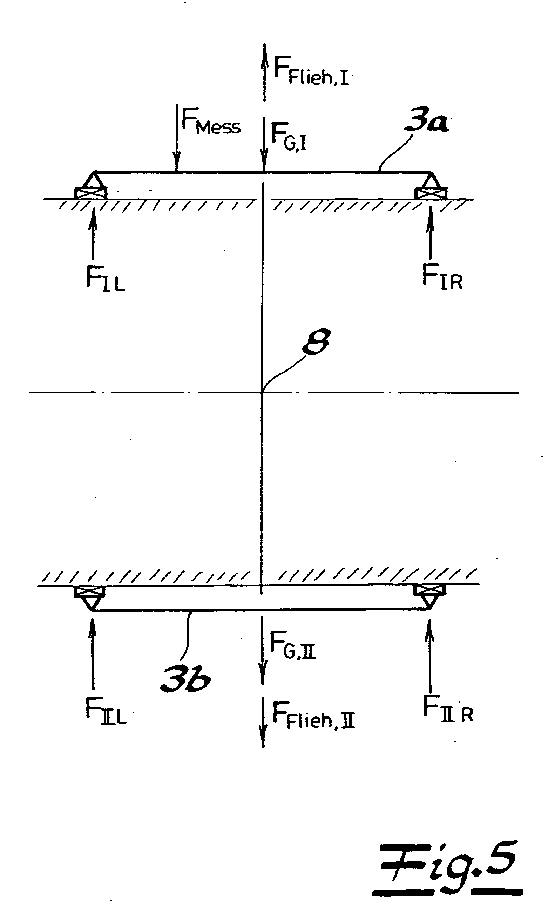

Die auf den Messkörper bzw. seine Teilmesskörper 3a, 3b wirkenden Kräfte in Z-Richtung sind in Fig. 5 dargestellt. Danach gilt

$$F_{G.I} = F_{G.II};$$

$$F_{Flieh,I} = F_{Flieh,II}$$

$$\sum F = 0 \rightarrow F_{Mess} = F_{IL} + F_{IR} + F_{IIL} + F_{IIR} - 2 F_{G,I}$$

Bezogen auf die Kraftsensoren $F_s$ gilt:

$$F_{S,IL} = F_{IL}; \; F_{S,IR} = F_{IR};$$

$$F_{S,IIL} = -F_{IIL}; \; F_{S,IIR} = -F_{IIR}$$

$$\rightarrow F_{Ness} = \sum F_{SI} - \sum F_{SII} - 2 F_{G,I}$$

**Patentansprüche**

1. Verfahren zur Ermittlung von Planheitsfehlern in Bändern (2), insbesondere Stahl- und Metallbändern, im Wege einer Messung der Bandzugspannungsverteilung über die Bandbreite, wobei das über die gesamte Bandbreite unter Zugspannung stehende Band eine Planheitsmessrolle (1) mit einem vorgegebenen Umschlingungswinkel umschlingt und **dadurch** örtliche Anpresskräfte entsprechend der örtlichen Längszugspannungsverteilung in Bandbreitenrichtung auf die Planheitsmessrolle (1) ausübt, aus denen sich die Bandzugspannungsverteilung erfassen lässt, wobei die Planheitsmessrolle (1) zumindest einen Messkörper (3) mit zwei um 180° versetzten, in den Rollenmantel (5) integrierten und auf Kraftmessgebern (4) abgestützten Teilmessgebern (3a, 3b) aufweist, die von dem Rollenmantel (5) durch einen umlaufenden Bewegungsspalt (6) entkoppelt und über zumindest eine Zugstange (7) miteinander verspannt sind, wobei ferner die Messwerte der Kraftmessgeber (4) eines jeden Teilmesskörpers (3a, 3b) aufsummiert werden, dann die Summenwerte der beiden Teilmesskörper (3a, 3b) voneinander subtrahiert werden und der **dadurch** gebildete Differenzmesswert der gesamten örtlichen Anpresskraft abzüglich der Gewichtskraft des Messkörpers (3) entspricht.

2. Verfahren nach Anspruch 1, **dadurch gekennzeichnet, dass** die Gewichtskraft des Messkörpers (3) in einer Kalibriermessung als Funktion des Rollendrehwinkels ohne Anpresskräfte ermittelt und als Korrekturfunktion für die Messwertauswertung herangezogen wird.

3. Planheitsmessrolle (1) zur Durchführung des Verfahrens nach Anspruch 1 oder 2, mit zumindest einem Messkörper (3) mit zwei um 180° versetzten in den Rollenmantel (5) integrierten und auf Kraftmessgebern abgestützten Teilmesskörpern, die von dem Rollenmantel (5) durch einen umlaufenden Bewegungsspalt (6) entkoppelt und über zumindest eine Zugstange (7) miteinander verspannt sind, wobei im Zuge einer Messung der Bandzugspannungsverteilung über die Bandbreite das über die gesamte Bandbreite unter Zugspannung stehende Band (2) die Planheitsmessrolle (1) mit einem vorgegebenen Umschlingungswinkel umschlingt und **dadurch** örtliche Anpresskräfte entsprechend der örtlichen Längszugspannungsverteilung in Bandbreitenrichtung auf die Planheitsmessrolle ausübt, aus denen sich die Bandzugspannungsverteilung ermitteln lässt.

4. Planheitsmessrolle nach Anspruch 3, **dadurch gekennzeichnet, dass** die Teilmesskörper (3a, 3b) kreisförmig ausgebildet und jeweils zentral auf einem Kraftmessgeber (4) abgestützt sind.

5. Planheitsmessrolle nach Anspruch 3, **dadurch gekennzeichnet, dass** die Teilmesskörper (3a, 3b) als Messbalken ausgebildet und in den Balkenendbereichen auf Kraftmessgebern (4) abgestützt sind.

6. Planheitsmessrolle nach einem der Ansprüche 3 bis 5, **dadurch gekennzeichnet, dass** die Kraftmessgeber (4) als Piezo-Quarze ausgebildet sind.

**7.** Planheitsmessrolle nach einem der Ansprüche 3, 5 oder 6, **dadurch gekennzeichnet, dass** die Messbalken gerade oder schräg zur Rollenachse (8) angeordnet sind.

**8.** Planheitsmessrolle nach einem der Ansprüche 3 bis 7, **dadurch gekennzeichnet, dass** in den die Teilmesskörper (3a, 3b) umgebenden Bewegungsspalt (6) ein dauerelastischer Kleber und/oder eine Dichtung eingesetzt ist.

**9.** Planheitsmessrolle nach einem der Ansprüche 3 bis 8, **dadurch gekennzeichnet, dass** die Messkörper (3) mehr als zwei Teilmesskörper (3a, 3b) aufweisen.

**10.** Planheitsmessrolle nach einem der Ansprüche 3 bis 9, **dadurch gekennzeichnet, dass** die Messkörper (3) bzw. ihre Teilmesskörper (3a, 3b) von einer Metallfolie abgedeckt sind.

**11.** Planheitsmessrolle nach einem der Ansprüche 3 bis 10, **gekennzeichnet durch** eine Ummantelung aus Gummi, Kunststoff oder Hartmetall.

**12.** Planheitsmessrolle nach einem der Ansprüche 3 bis 11, **dadurch gekennzeichnet, dass** zwei oder mehr Messkörper (3) im jeweiligen Bandbreitenbereich angeordnet sind.


## Claims

**1.** Method for determining flatness defects in strips (2), in particular steel and metal strips, by measurement of the strip tensile stress distribution over the strip width, wherein the strip, which is under tensile stress over the entire strip width, wraps around a flatness measuring roller (1) with a predefined wrap-around angle and as a result exerts on the flatness measuring roller (1) local pressing forces corresponding to the local longitudinal tensile stress distribution in the strip width direction, from which the strip tensile stress distribution can be determined, wherein the flatness measuring roller (1) has at least one measuring body (3) with two partial measuring bodies (3a, 3b) which are offset by 180° and are integrated in the roller outer surface (5) and are supported on force measuring heads (4), said partial measuring bodies being separated from the roller outer surface (5) by a circumferential movement gap (6) and being braced to one another by means of at least one tie rod (7), wherein furthermore the measured values of the force measuring heads (4) of a given partial measuring body (3a, 3b) are summed and then the summed values of the two partial measuring bodies (3a, 3b) are subtracted from one another and the differential measured value thus formed corresponds to the overall local pressing force minus the weight force of the measuring body (3).

**2.** Method according to Claim 1, **characterized in that** the weight force of the measuring body (3) is determined in a calibration measurement as a function of the angle of rotation of the roller without pressing forces, and is used as a correction function for the evaluation of the measured values.

**3.** Flatness measuring roller (1) for carrying out the method according to Claim 1 or 2, having at least one measuring body (3) with two partial measuring bodies which are offset by 180° and are integrated in the roller outer surface (5) and are supported on force measuring heads, said partial measuring bodies being separated from the roller outer surface (5) by a circumferential movement gap (6) and being braced to one another by means of at least one tie rod (7), wherein, during measurement of the strip tensile stress distribution over the strip width, the strip (2), which is under tensile stress over the entire strip width, wraps around the flatness measuring roller (1) with a predefined wrap-around angle and as a result exerts on the flatness measuring roller local pressing forces corresponding to the local longitudinal tensile stress distribution in the strip width direction, from which the strip tensile stress distribution can be determined.

**4.** Flatness measuring roller according to Claim 3, **characterized in that** the partial measuring bodies (3a, 3b) are circular and in each case are supported centrally on a force measuring head (4).

**5.** Flatness measuring roller according to Claim 3, **characterized in that** the partial measuring bodies (3a, 3b) are designed as measuring beams and are supported in the beam end regions on force measuring heads (4).

**6.** Flatness measuring roller according to any of Claims 3 to 5, **characterized in that** the force measuring heads (4) are designed as piezo quartzes.

**7.** Flatness measuring roller according to any of Claims 3, 5 or 6, **characterized in that** the measuring beams are arranged straight or at an incline with respect to the roller axis (8).

8. Flatness measuring roller according to any of Claims 3 to 7, **characterized in that** a permanently elastic adhesive and/or a seal is inserted into the movement gap (6) surrounding the partial measuring bodies (3a, 3b).

9. Flatness measuring roller according to any of Claims 3 to 8, **characterized in that** the measuring bodies (3) have more than two partial measuring bodies (3a, 3b).

10. Flatness measuring roller according to any of Claims 3 to 9, **characterized in that** the measuring bodies (3) or their partial measuring bodies (3a, 3b) are covered by a metal foil.

11. Flatness measuring roller according to any of Claims 3 to 10, **characterized by** a jacket made of rubber, plastic or hard metal.

12. Flatness measuring roller according to any of Claims 3 to 11, **characterized in that** two or more measuring bodies (3) are arranged in the respective strip width area.


**Revendications**

1. Procédé de détection de défauts de planéité dans des feuillards (2), en particulier des feuillards d'acier et autres bandes métalliques, au cours d'une mesure de la distribution des contraintes de traction dans le feuillard, sur sa largeur, le feuillard sous contrainte de traction sur toute sa largeur entourant un rouleau de mesure de planéité (1) avec un angle d'enroulement prédéfini et exerçant de ce fait, sur le rouleau de mesure de planéité (1), des forces de pression locales correspondant à la distribution locale des contraintes de traction longitudinales dans la direction de la largeur du feuillard à partir desquelles on peut détecter la distribution des contraintes de traction dans le feuillard, le rouleau de mesure de planéité (1) comportant au moins un corps de mesure (3) avec deux transmetteurs de mesure partiels (3a, 3b) décalés de 180°, intégrés à l'enveloppe du rouleau (5) et soutenus sur des transmetteurs de mesure de force (4), lesquels transmetteurs de mesure partiels sont désaccouplés de l'enveloppe du rouleau (5) par une fente de déplacement (6) périphérique et sont serrés entre eux par au moins un tirant (7), les valeurs de mesure des transmetteurs de mesure de force (4) de chaque corps de mesure partiel (3a, 3b) étant en outre additionnées, puis les valeurs additionnées des deux corps de mesure partiels (3a, 3b) sont soustraites les unes des autres et la valeur de mesure différentielle ainsi formée correspond à la force de pression locale totale diminuée de la force de poids du corps de mesure (3).

2. Procédé selon la revendication 1, **caractérisé en ce que** la force de poids du corps de mesure (3) est déterminée au cours d'une mesure d'étalonnage en tant que fonction de l'angle de rotation du rouleau sans forces de pression, et est utilisée comme fonction de correction pour l'exploitation des valeurs de mesure.

3. Rouleau de mesure de planéité (1) pour la mise en oeuvre du procédé selon la revendication 1 ou 2, comportant au moins un corps de mesure (3) avec deux corps de mesure partiels décalés de 180°, intégrés à l'enveloppe du rouleau (5) et soutenus sur des transmetteurs de mesure de force, lesquels corps de mesure partiels sont désaccouplés de l'enveloppe du rouleau (5) par une fente de déplacement (6) périphérique et sont reliés entre eux par au moins un tirant (7), au cours d'une mesure de la distribution des contraintes de traction dans le feuillard sur sa largeur, le feuillard (2), sous contrainte de traction sur toute sa largeur, entourant le rouleau de mesure de planéité (1) avec un angle d'enroulement prédéfini et exerçant ainsi des forces de pression locales correspondant à la distribution locale des contraintes de traction longitudinales dans la direction de la largeur du feuillard sur le rouleau de mesure de planéité, à partir desquelles on peut déterminer la distribution des contraintes de traction dans le feuillard.

4. Rouleau de mesure de planéité selon la revendication 3, **caractérisé en ce que** les corps de mesure partiels (3a, 3b) sont de forme cylindrique et sont soutenus chacun au centre sur un transmetteur de mesure de force (4).

5. Rouleau de mesure de planéité selon la revendication 3, **caractérisé en ce que** les corps de mesure partiels (3a, 3b) sont réalisés sous la forme d'une barre de mesure et sont soutenus sur des transmetteurs de mesure de force (4) dans les zones terminales de la barre.

6. Rouleau de mesure de planéité selon l'une des revendications 3 à 5, **caractérisé en ce que** les capteurs de mesure de force (4) sont réalisés sous la forme de cristaux piézo-électriques.

7.  Rouleau de mesure de planéité selon l'une des revendications 3, 5 ou 6, **caractérisé en ce que** les barres de mesure sont disposées en ligne droite ou obliquement par rapport à l'axe (8) du rouleau.

8.  Rouleau de mesure de planéité selon l'une des revendications 3 à 7, **caractérisé en ce que** dans la fente de déplacement (6), entourant les corps de mesure partiels (3a, 3b), est insérée une colle élastique permanente et/ou une garniture d'étanchéité.

9.  Rouleau de mesure de planéité selon l'une des revendications 3 à 8, **caractérisé en ce que** les corps de mesure (3) comportent plus de deux corps de mesure partiels (3a, 3b).

10. Rouleau de mesure de planéité selon l'une des revendications 3 à 9, **caractérisé en ce que** les corps de mesure (3) ou leurs corps de mesure partiels (3a, 3b) sont recouverts par une feuille métallique.

11. Rouleau de mesure de planéité selon l'une des revendications 3 à 10, **caractérisé par** un gainage en caoutchouc, matière plastique ou métal dur.

12. Rouleau de mesure de planéité selon l'une des revendications 3 à 11, **caractérisé en ce que** deux corps de mesure (3) ou plus sont disposés dans la zone respective de la largeur du feuillard.

Fig.1

*Fig.2*

Fig.3

EP 1 566 227 B1

*Fig.4*

Fig.5